# EUROPEAN PATENT APPLICATION

(11) **EP 4 310 192 A1**
(43) Date of publication of application: **24.01.2024**
(21) Application number: 22306091.4
(22) Date of filing: 21.07.2022
(51) Int. Cl.: C12Q 1/18

(54) **METHOD OF DETERMINING THE EXISTENCE AND/OR DEGREE OF RESISTANCE OF MICROORGANISMS TO ANTIMICROBIAL AGENTS**

(71) Applicant: C4Diagnostics, 13013 Marseille (FR)
(72) Inventor: LAZRAK, Younes, 13006 MARSEILLE (FR)
(74) Representative: Santarelli

(57) **Abstract**

The present invention proposes a method of determining the existence and/or degree of resistance of a microorganism to one or more antimicrobial agents.

## Description

### TECHNICAL FIELD

The present invention belongs to the general field of microbiology and more particularly to the field of qualitatively / quantitatively determining the antimicrobial susceptibility of a microorganism, in particular a bacteria.

The present invention proposes a method of determining the existence and/or degree of resistance of a microorganism to one or more antimicrobial agents. The invention also relates to a more global method of analysing a sample for its microbial characteristics, comprising determining the existence and/or degree of resistance of a microorganism to one or more antimicrobial agents by a such method as of the invention.

### PRIOR ART

In the field of medicine, in the food, biotechnological and pharmaceutical industries, for military and civilian defence, and environmental control, the repeated and inappropriate use of antibiotics has favoured the appearance of microorganisms resistant to these molecules. The consequence of this inappropriate use is that it is increasingly difficult to fight against the infection by some microorganisms, in particular by some bacteria.

In particular, in the therapeutic field, when a microorganism is detected in a patient's biological sample, it is desirable to determine to which antibiotic(s) this microorganism is susceptible. There are now a high number of bacterial species, which increasingly exhibit resistance to one or more classes of antimicrobial agents, making it all the more important to perform susceptibility to antibiotics testing in order to better target which antibiotic should be best administrated to the patient.

This is for example the case for the treatment of urinary tract infection (UTI), which requires microbial analysis of a patient's urine.

UTI is a general term for infectious diseases in various parts of the urinary system.

Symptoms from a lower urinary tract include pain with urination, frequent urination, and feeling the need to urinate despite having an empty bladder. Symptoms of a kidney infection include fever and flank pain usually in addition to the symptoms of a lower UTI. In some cases, the urine may appear bloody. In the very old and the very young, symptoms may be vague or non-specific. A common cause of infection is the presence of pathogenic *Escherichia coli* in the urinary tract, though other bacteria, viruses or fungi may be the cause.

UTI is a common infection that is usually treated by antibiotics and can result in serious complications, such as renal failure. In order to avoid these complications as well as overuse of antibiotics, efficient and well-targeted treatment is particularly important.

Methods have been proposed by the prior art for determining the susceptibility of a microorganism to antibiotics, and antimicrobial agents in general. However, these methods are time-consuming, costly and/or lack sensitivity, and none of them is entirely satisfactory.

Thus, there remains a need for a method of determining the existence and/or degree of resistance of a microorganism to an antimicrobial agent, which is sensitive and rapid to implement, at low cost. The invention aims at providing such a method. In particular, an objective of the invention is that this method can be carried out without using any expensive device, in particular any expensive optical device.

### DISCUSSION OF THE INVENTION

The present invention proposes a method of determining the existence and/or degree of resistance of a microorganism to antimicrobial agents, using universal labelling, i.e. a fluorescent compound and a membrane having an upper surface and an opposite lower surface which have clearly defined different functions. Indeed, the microorganism is deposited on the upper surface and develop thereon, while the lower surface is the one in contact successively with a culture medium, a fluorescent compound and washing solution(s). Another advantage of the present invention is that no fixation is requested to scan the microorganism labelled with the fluorescent compound. This lack of fixation allows to repeat the steps of labelling with the fluorescent compound, of washing and of scanning as many times as necessary.

Detection methods using a fluorescent compound are well-known in the art but, as already mentioned, a fixation step is requested and they are often used on microorganisms in the form of single cells with the fluorescent compound being deposited onto the microorganisms. The present invention implements microorganism in the form of colonies and/or cell layers on the upper surface of a membrane and a fluorescent compound put into contact with the microorganisms via the lower surface of this membrane. It should be noted that, before the present invention, it was not at all obvious that what was realized on single cells would work on colonies and that the fluorescent compound would be able, during the short contact time, to diffuse uniformly and to be sufficient for an observation without requesting sophisticated and expensive equipment.

In addition, thanks to the labelling of the microorganism and the washing via the lower surface of the membrane, there is no risk of eliminating microorganism colonies and the fixation is not necessary.

In the present application, all the ranges must be interpreted as limits included. It is an object of the present invention to provide a method of determining the existence and/or degree of resistance of a microorganism to one or more antimicrobial agents, each at one or more concentrations, comprising successive steps of:
a. forming a suspension of said microorganism in a liquid medium,
b. depositing homogeneously microorganism cells contained in said suspension on at least a part of the upper surface of a membrane,
c. laying said membrane by its lower surface on a culture medium,
d. placing one or more substrates each impregnated with one of said antimicrobial agents in contact with said membrane,
e. incubating the whole under conditions suitable for promoting the growth of said microorganism,
f. putting the lower surface of said membrane into contact with a fluorescent compound capable of labelling an endogenous constituent of said microorganism in order to label said microorganism cells,
g. optionally washing the lower surface of said membrane thereby eliminating the excess of fluorescent compound,
h. detecting the presence or absence of fluorescence around the substrate(s) on the upper surface of the membrane using a fluorescence-reading optical device,
i. and determining the existence and/or degree of resistance of said microorganism to said antimicrobial agent(s) based on the detection of presence and/or size of a non-fluorescent zone around said substrate(s).

By "resistance", it is meant the ability of a microorganism to resist the effects of the antimicrobial agent(s).

By "microorganism", it is meant a microscopic organism, especially a bacteria, virus, or fungus.

The microorganism whose sensitivity to one or more antimicrobial agents is tested according to the method of the present invention are organisms and notably unicellular organisms of the eukaryotic type or organisms and notably unicellular organisms of the prokaryotic type.

The microorganisms of the eukaryotic type may be yeasts such as yeasts of the genus Saccharomyces or Candida like *Candida albicans,* fungi, algae or mixtures thereof.

The microorganisms of the prokaryotic type are bacteria, which may be of the Gram positive type or Gram negative type, archaea or mixtures thereof.

Thus, the microorganism whose sensitivity to one or more antimicrobial agents is tested according to the method of the present invention is advantageously selected from yeasts, fungi, algae, bacteria of the Gram positive type, bacteria of the Gram negative type, archaea and mixtures thereof.

Among the bacteria, mention may be made, as an example and in a non-exhaustive way, of bacteria belonging to branches of spirochetes and chlamydiae, of bacteria belonging to the families or genera of Alcaligenaceae, Pasteurellaceae, Enterobacteriaceae, Enterococcus, Staphylococcus, Pseudomonadaceae, Streptococcus, Micrococci, Legionellaceae, Mycobacteria, Bacillaceae and Cyanobacteria. More particularly, these bacteria may be selected from the group consisting of *Bordetella pertussis, Haemophilus influenza, Citrobacter freundii, Escherichia coli, Enterococcus faecalis, Klebsiella pneumoniae, Klebsiella aerogenes, Legionella pneumophila, Morganella morganii, Pseudomonas aeruginosa, Proteus mirabilis, Serratia marcescens, Bacillus anthracis, Staphylococcus aureus, Staphylococcus saprophyticus* and *Streptococcus agalactiae.*

Preferably, said microorganism is *Escherichia coli, Pseudomonas, Candida* and/or *Staphylococcus.*

From among archaea, as examples and in a non-exhaustive way, mention may be made of the archaea belonging to the phyla of Crenarchaeotes and Euryarchaeotes.

Of course, the method of the invention can be applied to a mixture of microorganisms. Therefore, in the present description, the term "microorganism" also encompasses a mixture of microorganisms, the global susceptibility to antimicrobial agent(s) thereof being then assessed by the method of the invention.

By "antimicrobial agents", it is meant a natural or synthetic substance that kills or inhibits the growth of microorganisms such as bacteria, fungi and algae.

Among the antimicrobial agents, mention may be made, as an example and in a non-exhaustive way, of antibacterials, antifungals, antivirals, antiparasitics, broad-spectrum therapeutics, non-pharmaceutical antimicrobial agents (such as essential oils, and antimicrobial pesticides), ozone, antimicrobial scrubs, halogens, alcohols, phenol and phenolic compounds.

Preferably, the antimicrobial agent according to the present invention is antibacterial.

In preferred embodiments of the invention, at least one, preferably all, antimicrobial agents are antibiotics.

In step (a) according to the present invention, a suspension of microorganism in a liquid medium is formed.

Any method suitable therefore can be implemented according to the invention. As an example, the microorganism can be transferred from its original medium to a liquid medium.

By "original medium", it is meant any sample, liquid or solid, that may contain a microorganism by nature of following a contamination.

Preferably, the original medium according to the invention is a liquid sample.

Typically, this liquid sample can be a biological fluid; a plant fluid such as sap, nectar and root exudate; a sample in a culture medium or in a biological culture reactor such as a cell culture of higher eukaryotes, of yeasts, of fungi or of algae; a liquid obtained from one (or more) animal or plant cell(s); a liquid sample obtained from an animal or plant tissue; a liquid sample obtained from a food matrix; a liquid sample from a chemical reactor; municipal, river, pond, lake, sea, or air-cooled tower water; a sample from a liquid industrial effluent; waste water coming in particular from intensive animal production or industries of the chemical, pharmaceutical, cosmetic or nuclear field; a liquid sample from a pharmaceutical product; a liquid sample from a cosmetic product; a fragrance; a soil sample or a mixture thereof.

Within the scope of the present invention, the sample may be obtained by any type of sample collection, for example, by contact, scraping, drilling, draining, washing, rinsing, suction, pumping, etc...

When the liquid sample is a biological fluid, the latter is advantageously selected from the group consisting of blood such as whole blood or anti-coagulated whole blood, blood serum, blood plasma, lymph, saliva, sputum, tears, sweat, sperm, urine, faeces, milk, cerebrospinal fluid, interstitial fluid, a fluid isolated from bone marrow, a mucus or fluid isolated from respiratory tract, intestinal tract or genito-urinary tract, cellular extract, tissue extract and organ extract. Thus, the biological fluid can be any fluid naturally secreted or excreted from a human or animal body or any fluid recovered, from a human or animal body, by any technique known to those skilled in the art such as extraction, sampling or washing. The steps of recovering and isolating these different fluids from the human or animal body are made prior to the implementation of the method according to the present invention. Preferably, the liquid sample is urine.

Likewise, if one of the contemplated samples does not allow implementing the method according to the present invention, for example because of its nature being in particular solid, its concentration or elements it contains such as solid residues, waste, suspension or interfering molecules, the sampling such as defined hereinafter further comprises a prior step of preparing the sample with optionally solubilizing the sample by techniques known to those skilled in the art such as filtration, precipitation, dilution, distillation, mixing, concentration, lysis, etc.

For example, when the liquid sample is urine, the latter may be filtrated in order to remove interfering substances such as crystals, hyaline structure, cell aggregates and/or big eukaryotic cells. To this extend, a sterile sieving device with a porosity of 5 µm or 10 µm such as a pluriStrainer^{®} can be used. Nevertheless, this filtration is optional.

Preferably, the suspension of microorganism in a liquid medium formed according to the invention has a McFarland value of between 0.4 and 0.6.

By "McFarland value", it is meant a reference to standardize the approximate number of bacteria in a liquid suspension by comparing the turbidity of the test suspension with that of the McFarland Standard. The turbidity of a microorganism suspension being proportional to the number of microorganism cells in the suspension, the number of cells in the suspension will be within an associated range to standardize microbial testing.

Preferably, the McFarland value of the suspension of step (a) according to the invention is around 0,5.

In particular embodiments of the invention, the liquid medium is a nutritive medium for the microorganism, such as liquid lysogeny broth (LB) medium or Mueller Hinton broth (MH), or saline solution (NaCl 0.9%), and said medium is inoculated with the microorganism and incubated in conditions promoting the growth of the microorganism, until a sufficient number of cells is reached to obtain the desired McFarland value.

Any other means of forming a microbial suspension with a McFarland value of between 0.4 and 0.6 falls within the scope of the invention, for example the dilution of overly concentrated cell suspension already available, for example from a prior step of detecting and/or identifying microorganisms in an original sample.

By "membrane", it is meant a thin wall of a porous substance that is interposed between two media and that allows the removal or concentration of certain constituents.

It is clear that the membrane implemented in the present invention is able to retain the microorganism contained in the suspension and/or is impermeable to this microorganism. Advantageously, this membrane is porous with naturally occurring pores or artificially created pores and with a porosity suitable for retaining the microorganism present in the suspension. Advantageously, this porosity is less than 0.5 µm and in particular of 0.45 µm or less.

Any membrane usually implemented for filtering a liquid sample and retaining the microorganisms present therein may be used within the present invention. Suitable membranes for use in the present invention include, without limitation, membranes made of polytetrafluoroethylene (PTFE), polyester, polycarbonate, nylon, polyvinylidene fluoride (PVDF), cellulose and cellulose derivatives, such as cellulose acetate, cellulose nitrate, regenerated cellulose, nitrocellulose, cellophane, or mixed cellulose esters (MCE). Such membranes are in particular available from the companies Whatman, Merck Millipore, and Sigma Aldrich.

Advantageously, the membrane implemented in the present invention is a MCE membrane, in particular a black MCE membrane and, more particularly, the black MCE membrane No. 1 sold by Merck Millipore.

It should be noted that the membrane implemented in the present invention does not necessarily have to be subjected to a pre-treatment before step (b). This pre-treatment may be a coating and in particular a coating with microbe-binding molecules as defined in [1].

The step (b) of the method according to the present invention comprise depositing homogeneously microorganism cells contained in said suspension on at least a part of the upper surface of a membrane. In this step, the membrane may or may not be already laying by its lower surface on a culture medium.

Such a deposition step can for example be performed by filtering at least part of the suspension on the membrane, so as to collect the microorganism cells thereon. In such embodiments, it is preferable for the membrane not yet to lay on the culture medium. It can otherwise for example be performed by depositing a small volume of the suspension on the membrane and spreading the deposited cells homogeneously thereon using beads or any other conventional means. In such embodiments, it is preferable for the membrane to already lay on the culture medium.

By "culture medium", it is meant a substance containing nutrients in which microorganisms can be grown.

In a first embodiment, the culture medium implemented at step (c) of the method according to the present invention is a liquid medium.

This liquid culture medium implemented at step (c) of the method according to the present invention may be any liquid medium suitable for culturing microorganisms, known by those skilled in the art. The latter has a carbon source, such as glucose or glycerol; a nitrogen source, such as ammonium or nitrate or amino acids; and salts and/or trace elements and vitamins for the growth of microorganisms. In particular, this liquid culture medium may not be selective to a particular type of microorganisms. Alternatively, it may be selective to a particular type of microorganisms.

As examples of liquid media suitable for using at step (c) of the method according to the present invention, one can cite Nutrient Broth No. 1 (NB1), Nutrient Broth No. 2 (NB2), Mueller Hinton broth, Lysogenic Broth (LB) (also known as Luria Bertani), Tryptic SoyBean (TSB) culture liquid and Brain Heart Infusion (BHI) broth. Such culture media are in particular available from the companies ThermoFisher, Bio-Rad Laboratories and Sigma Aldrich.

These different liquid culture media may be supplemented by one or more elements selected in the group consisting of an antioxidant agent such as sodium pyruvate, glutamate, calcium chloride and magnesium chloride; a multi-vitamin such as Polyvitex^{®} (PVX); a source of iron such as iron citrate or Vitox^{®}; a protein supplement such as Bovine Serum Albumin (BSA); and an anti-clumping agent such as citrate salt.

Advantageously, the liquid culture medium is sterile or has been sterilized before contacting the lower surface of the membrane at step (c) of the method according to the present invention. The culture medium can be sterilized by various techniques known in the art, such as, but not limited to, the use of aseptic techniques to autoclave and/or prepare the culture medium.

In a second embodiment, the culture medium implemented at step (c) of the method according to the present invention is a solid culture medium.

In this second embodiment, the solid culture medium may be a diffusion intermediate impregnated with a liquid culture medium such as previously defined. A diffusion intermediate is a solid support which has to be porous in order to allow good impregnation with the liquid culture medium such as previously defined. Advantageously, the diffusion intermediate is a porous solid support such as a pad in a material selected from the group consisting of paper notably of a cellulose nature; cotton paper; agarose; gelatin; cellulose; methylcellulose; carboxymethylcellulose; nitrocellulose; cellulose acetate ester; an alginate; a polyolefin; a porous membrane and notably an ion exchange porous membrane; a resin of the sephadex type packaged as a membrane or a membrane of a perfluorinated polymer such as PVDF; a felt fabric; a glass fiber fabric; a membrane in an organic polymer such as polyethylene, polypropylene or mixtures thereof; a nylon fabric; a polyacrylamide gel; a sepharose gel and one of their mixtures.

As a variant of this second embodiment, the solid culture medium may be a nutritive gel. The latter may be any nutritive gel suitable for culturing microorganisms, known by those skilled in the art. As for liquid culture medium, this nutritive gel has a carbon source, such as glucose or glycerol; a nitrogen source, such as ammonium or nitrate or amino acids; and salts and/or trace elements and vitamins for the growth of microorganisms. In particular, this solid culture medium may not be selective to a particular type of microorganisms. Alternatively, it may be selective to a particular type of microorganisms.

As examples of solid media suitable for using at step (c) of the method according to the present invention, one can cite nutrient agar, Tryptic Soy Agar (TSA), Mueller Hinton agar, Legionella Glycine Vancomycine Polymyxine Cycloheximide (GVPC) agar, buffered charcoal yeast extract (BCYE) agar, Buffered cefaMandole Polymyxine Anisomycine α-cetoglutarate (BMPAα) agar, Reasoner's 2A (R2A) agar, Sabouraud Dextrose Agar (SDA), Columbia agar with horse blood, blood agar such as sheep blood agar, chocolate agar with Vitox, Haemophilus Test Medium (HTM) agar and charcoal agar. Such culture media are in particular available from the companies ThermoFisher, Oxoid and VWR.

Advantageously, when the culture medium implemented at step (c) of the present invention is solid, it is blood agar.

In addition, it is possible, when using a solid culture medium, to add some liquid culture medium such as previously defined between the solid culture medium and the membrane, on the upper surface of which are the microorganisms. Nevertheless, this addition is optional.

The step (d) of the method according to the present invention comprises placing one or more substrates each impregnated with one of said antimicrobial agents on the part of the upper surface of the membrane on which the microorganism cells have been deposited.

By "substrate", it is meant any support capable of at least partially absorbing an antimicrobial agent, and diffusing it in its environment.

In one embodiment, step d is performed before step b or before step c. In this case, the antimicrobial agent diffuses from the substrate through the membrane to the surface of the membrane

In another embodiment, step d is performed after step c. In this case, the antimicrobial agent diffuses from the substrate onto the upper surface of the membrane to form an antimicrobial gradient thereon.

An inhibition of the growth of the microorganism on the membrane, around the substrate, during subsequent step (e) of the method of the invention, denotes a susceptibility of this microorganism to this antimicrobial agent. The larger the growth inhibition zone, the lower the degree of resistance of the microorganism to this antimicrobial agent. The absence of any growth inhibition zone on the membrane denotes a resistance of the microorganism to the tested antimicrobial agent.

The method according to the invention can use a single substrate, for assessing the susceptibility of the microorganism to a single antimicrobial agent impregnating the substrate. It can otherwise use a plurality of substrates, each impregnated with a different antimicrobial agent, for assessing the susceptibility of the microorganism to a said plurality of antimicrobial agents. In such embodiments of the invention, the substrates are preferably placed well apart from one another on the membrane so as to interfere with one another, as recommended by the laboratory testing guidelines.

The substrates implemented according to the present invention are either antibiotic-impregnated discs or elongated strips impregnated with a gradient of antibiotics (Etest^{®} strips).

In particular embodiments of the method according to the present invention, at least one substrate, preferably all substrates, are disks each impregnated with a single concentration of an antimicrobial agent.

In embodiments of the method according to the present invention, at least one substrate, preferably all substrates, are strips impregnated with concentration gradient of an antimicrobial agent along its length.

Advantageously, the substrates implemented according to the present invention are made of paper or plastic. Preferably, they are made of plastic.

Preferably, the substrates implemented according to the present invention are strips impregnated with a gradient of antibiotics, for example such as those marketed under the brand name Etest^{®}). Said strips are made of a thin, inert, non-porous plastic slide with a dilution range following a predefined antibiotic gradient on one side and a MIC reading scale in µg/mL on the other side.

The step (e) of the method according to the present invention comprises incubating the whole under conditions suitable for promoting the growth of said microorganism, i.e., the microorganism is maintained in an environment that allows it to increase in number through cell division in order to form microcolonies, colonies, or even layers of cells. This environment includes not only the culture medium put into contact with the lower surface of the membrane but also other conditions such as a temperature and an atmosphere that permit cell growth. Typically, the temperature implemented during step (e) is comprised between 25°C and 40°C. Particular examples of temperature implemented at step (e) are 30°C, 32°C, 37°C and 39°C. Typically, concerning the atmospheric conditions, the step (e) may be performed under air, under air + 5% CO₂ or under a gaseous mixture consisting of 5% O2, 5% CO₂ and 90% N₂.

Typically, depending on the type of microorganisms present in the liquid sample, the step (e) has to last no more than 8 h, notably no more than 7 h, in particular no more than 6 h and more particularly no more than 5 h. Indeed, as for example, from the results obtained by the inventors, in particular, in the conditions as disclosed in the hereinafter experimental part, colonies of *E. coli* or of S. *aureus* can be detected after 4 h of incubation with the culture medium.

In particularly advantageous embodiments of the invention, the suspension formed in step (a) has a McFarland value of between 0.4 and 0.6 and the incubating step (e) is carried out for a duration of between 3 and 6 hours.

The step (f) of the method according to the present invention is a labelling step in order to label with a fluorescent compound the microorganism cells that have grown onto the upper surface of the membrane obtained after steps (b) to (e).

Generally, the term "fluorescent compound" refers to a compound capable of emitting light when excited by another light of appropriate wavelength. The term "fluorescent", as applied to a compound, can be used to refer to its property of absorbing energy (such as UV, visible or IR radiation) and re-emitting at least a fraction of that energy as light over time.

The present invention implements a fluorescent compound capable of recognizing and binding an endogenous constituent of the microorganism. By definition, an endogenous constituent means a constituent that originates from within the microorganism.

Typically, the fluorescent compound implemented at step (f) of the method according to the present invention is capable to recognize and bind, and thus to label, mitochondria, cell membranes or nucleic acids.

In a first embodiment, the fluorescent compound implemented at step (f) of the method according to the present invention is capable of recognizing and binding, and thus labelling, mitochondria. Advantageously, this fluorescent compound is selected from the group consisting of Rhodamine123, DiOC6(3), DiOC7(3), JC-1, MitoTracker Orange and Red CMXRos.

In a second embodiment, the fluorescent compound implemented at step (f) of the method according to the present invention is capable of recognizing and binding and, thus labelling, cell membranes. Advantageously, this fluorescent compound is selected from the group consisting of FM1-43 and FM4-64.

In a third embodiment, the fluorescent compound implemented at step (f) of the method according to the present invention is capable of recognizing and binding, and thus labeling, nucleic acids. "Nucleic acid" is understood to mean a single-stranded or doublestranded desoxyribonucleic acid (DNA), a ribonucleic acid (RNA) such as a messenger RNA or a ribosomal RNA.

The fluorescent compounds specific for nucleic acids are in particular selected from the fluorescent intercalating agents, dyes binding to the bases A:T or the bases G:C and the permeating or non-permeating cyanines. More particularly, said fluorescent compounds are selected from the group consisting of ethidium bromide, thiazole orange, thiazole blue and derivatives thereof, thioflavin S, thioflavin T, thioflavin TCN, diethylquinolylthio-cyanine iodide (DEQTC), TOTO1, TOPRO1, TOTO3, TOPRO3, YOYO1, Hoechst 33258, Hoechst 33342, Hoechst 34580, 4',6-diamidino-2-phenylindole (DAPI), pyronin Y, acridine orange, auramine O, calcein, olamin-O, Oxazine 750, Astra blue and the SYTO series comprising in particular SYTO 11, SYTO 12, SYTO 13, SYTO 15, SYTO 16, SYTO 18, SYTO 62, SYTO 80 or SYTO 81.

The fluorescent compound can be selected in the group consisting of 4',6-diamidino-2-phenylindole (DAPI), Rhodamine123, DiOC6(3), DiOC7(3), JC-1, MitoTracker Orange, Red CMXRos, FM1-43, FM4-64, thiazole orange, thiazole blue and derivatives thereof, TOTO1, TOPRO1, TOTO3, TOPRO3, YOYO1, Hoechst 33258, Hoechst 33342, Hoechst 34580, acridine orange, calcein, olamin-O, Oxazine 750, Astra blue and the SYTO series comprising in particular SYTO 11, SYTO 12, SYTO 13, SYTO 15, SYTO 16, SYTO 18, SYTO 62, SYTO 80 or SYTO 81.

In a particular embodiment, the fluorescent compound implemented at step (f) of the method according to the present invention is 4',6-diamidino-2-phenylindole (DAPI).

During this step (f), the fluorescent compound is put into contact with the lower surface of the membrane, which thus is to be permeable thereto.

In a particular embodiment, the lower surface of the membrane may be brought into contact with a solution or dispersion of the fluorescent compound. The fluorescent compound is preferably present in the solution or dispersion in an excess with respect to the microorganisms that are present on the upper surface of the membrane.

In another particular embodiment, the lower surface of the membrane may be brought into contact with a solution or dispersion of the fluorescent compound such as previously defined via a diffusion intermediate impregnated with said solution or dispersion. A diffusion intermediate is as previously defined for the solid culture medium provided that, in step (e), this diffusion intermediate is a solid support which has to be porous in order to allow good impregnation with the solution or dispersion of the fluorescent compound. A particular example of such a diffusion intermediate useful in step (f) is a glass fiber fabric impregnated with a solution or dispersion of a fluorescent compound such as a DAPI solution and more particularly a solution comprising 1.4 µg/ml of DAPI.

Typically, the duration of step (f) of the method according to the present invention is equal to or below 30 min, advantageously equal to or below 25 min, notably equal to or below 20 min, in particular, equal to or below 15 min and more particularly of 10 min (i.e. 10 min ± 2 min).

The step (f) of the method according to the present invention may be carried out at a temperature of between 10°C and 40°C, advantageously between 15°C and 30°C and more particularly at room temperature (i.e. 23°C ± 5°C).

In particular, the step (f) of the method according to the present invention can be performed in the dark.

The step (g) of the method according to the present invention is an optional washing step, which allows the removal of any fluorescent compound present on the lower surface of the membrane. Indeed, the presence of this fluorescent compound adsorbed on the lower surface of the membrane might generate a background noise during the step (h).

The lower surface of the membrane is subjected to at least one rinsing in order to remove all traces of the fluorescent compound and/or of the solution or dispersion containing it. This rinsing is typically performed with a rinsing solution and, in particular, an aqueous rinsing solution that does not affect the microorganism cells on the upper surface of the membrane. This solution may also help eliminate non-specific interactions. It may comprise one or more of the following components: a buffer such as a Tris, phosphate, acetate or borate buffer; salts such as KCI, NaCl, (NH₄)₂SO₄, MgCl₂ or CaCl₂; and detergents or surfactants such as Tween^{®}, Triton^{®}, sodium dodecyl sulfate (or SDS) or serum bovine albumin (BSA). This rinsing may be repeated twice, three times, five times, 10 times and even 50 times using, for each rinsing, an identical or different rinsing solution. Typically, the rinsing is repeated three times with a solution comprising phosphate buffered saline (or PBS) and 0.1% Tween^{®} and having a pH comprised between 7 and 7.5.

Advantageously, this washing step (g) may be performed by continuously flowing the rinsing solution at the level of the lower surface of the membrane. This may be implemented using a washing device for membranes well-known in the field such as the washing module sold by DIAMIDEX^{®}.

Typically, the duration of step (g) of the method according to the present invention is equal to or below 40 min, advantageously equal to or below 30 min, notably equal to or below 20 min, in particular, of 15 min (i.e. 15 min ± 3 min).

The step (g) of the method according to the present invention may be carried out at a temperature of between 10°C and 40°C, advantageously between 15°C and 30°C and in particular at room temperature (i.e. 23°C ± 5°C).

In particular, the step (g) of the method according to the present invention is performed in the dark.

The step (h) of the method according to the present invention consists in placing the membrane obtained after step (g) under an optical device capable of reading fluorescence, such as a microscope stage where it can be interrogated by an excitation source adapted to the fluorescent compound implemented.

In an embodiment of the method implemented according to the present invention, the images of the membrane are captured at one or more intervals with at least one image capture device.

Typically, the excitation source results in the excitation of the fluorescent compound labelling the microorganism, followed by measurement of the emission of fluorescence from this compound.

The membrane can be manually manipulated to position the substrates of step (d) for interrogation, either by moving the membrane itself or moving the optical device on which the membrane is placed. Alternatively, the optical device is automatically controlled such that the membrane on the stage can be scanned.

Light sources capable of emission in the ultraviolet, visible and/or near-infrared spectra well-known to those skilled in the art may be used in the present invention. For example, light sources may be continuum lamps such as a deuterium or xenon arc lamp for generation of ultraviolet light and a tungsten halogen lamp for generation of visible/near-infrared excitation. Alternatively, the light source can be one or several LED(s). These light sources provide a broad emission range and the spectral bandwidth for specific excitation wavelengths may be reduced using filters such as optical interference filters, prisms and/or optical gratings.

Therefore, in particular embodiments of the invention, a light source in said optical device comprises one or several LED(s).

The one skilled in the art will know which excitation source to be used depending on the fluorescent compound implemented in the method according to the present invention. Advantageously, the excitation source implemented at step (h) of the method according to the present invention is an optical microscope equipped with filters such as UV filter or Cy5 filter.

The measurement data obtained while performing step (h) may be analysed by algorithms and in particular compared with control measurements.

Typically, the duration of step (h) of the method according to the present invention is equal to or below 20 min, advantageously equal to or below 15 min, notably equal to or below 10 min, in particular, of 7 min (i.e. 7 min ± 2 min).

Taking into account of the duration of the different steps of the method according to the present invention, steps (b) to (i) of the latter are performed in less than 10 h in particular, in less than 9 h, preferably, in less than 8 h, more preferably, in less than 7 h, even more preferably, in less than 6 h, and even more preferably, in less than 5 h.

The optical device involved in step (h) of the present invention is a simple inexpensive optical device.

In an embodiment, the optical device of step (h) has a magnification between 1 and 50 and an optical resolution comprised between 6-20 Mpix.

Preferably, this optical device has a magnification between 1 and 8, more preferably between 2 and 4.

Preferably, the optical resolution of the optical device is comprised between 9 and 15, more preferably, between 11-13 Mpix.

The existence and/or degree of resistance of said microorganism to said antimicrobial agent(s) is determined in step (i) of the method of the invention, based on the detected absence, or presence, and optionally size, of a non-fluorescent zone around said substrate(s). It is within the skills on the person skilled in the art to determine, based on the size of the growth inhibition zone, the degree of resistance of the microorganism to the antimicrobial agent.

In an embodiment, the method according to the present invention comprises the determination of a minimum inhibitory concentration (MIC) of the antimicrobial agent for the microorganism.

By "minimum inhibitory concentration (MIC)", it is meant the lowest concentration of a chemical, usually a drug, which prevents visible growth of the targeted microorganism.

MIC depends on the microorganism and on the antimicrobial agent itself. It is often expressed in micrograms per milliliter (µg/mL) or milligrams per liter (mg/L).

Advantageously, in the method of the present invention, the MIC is assessed using a strip containing a predefined and continuous gradient of antibiotic concentrations, for example an E-test^{®} or equivalent.

Preferably, the strip is graduated and indicates the concentrations of antibiotics present at all heights of the strip, which facilitates the interpretation of the results.

To obtain an inhibition ellipse for determining MIC, according to the invention the strip is simply placed on the seeded membrane and incubated. After the incubation period, the MIC value (in µg/mL) can be read directly on the scale at the intersection between the inhibition ellipse and the strip. This MIC value is indicative for the skilled person who will then be able to select the antimicrobial agent and its amount for an optimal treatment. Indeed, the MIC value read on the strip is compared to the critical concentrations defined by the (European Committee on Antimicrobial Susceptibility Testing (EUCAST) for the pathogen/antibiotic pair. If the MIC value read on the strip is lower than the minimum critical concentration, then the pathogen is sensitive to the antibiotic at standard dosage; if the MIC value read on the strip is higher than the maximum critical concentration, then the pathogen is said to be resistant to the antibiotic; finally, if the MIC value read on the strip is between the minimum and maximum critical concentration, then the pathogen is said to be intermediate or sensitive to the antibiotic at high dosage.

Another aspect of the invention concerns a method of analysing a sample for its microbial characteristics, said method comprising:
- detecting the presence of at least one microorganism in said sample,
- optionally, identifying said microorganism,
- recovering said microorganism from said sample,
- and performing, on said recovered microorganism, a method according to the invention of determining the existence and/or degree of resistance of a microorganism to one or more antimicrobial agents, each at one or more concentrations.

The steps of detecting the presence of at least one microorganism in the sample, and of optionally identifying said microorganism, can be carried out by any methods known in the art.

The sample can be any of a biological fluid; a plant fluid such as sap, nectar and root exudate; a sample in a culture medium or in a biological culture reactor such as a cell culture of higher eukaryotes, of yeasts, of fungi or of algae; a liquid obtained from one (or more) animal or plant cell(s); a liquid sample obtained from an animal or plant tissue; a liquid sample obtained from a food matrix; a liquid sample from a chemical reactor; municipal, river, pond, lake, sea, or air-cooled tower water; a sample from a liquid industrial effluent; waste water coming in particular from intensive animal production or industries of the chemical, pharmaceutical, cosmetic or nuclear field; a liquid sample from a pharmaceutical product; a liquid sample from a cosmetic product; a fragrance; a soil sample or a mixture thereof. It can respond to any of the features described above regarding the original medium containing the microorganism to be assayed.

Other characteristics and advantages of the present invention will additionally be apparent to the one skilled in the art on reading the examples below, which are given as an illustration and not a limitation, with reference to the attached figures.

### LEGENDS OF THE FIGURES

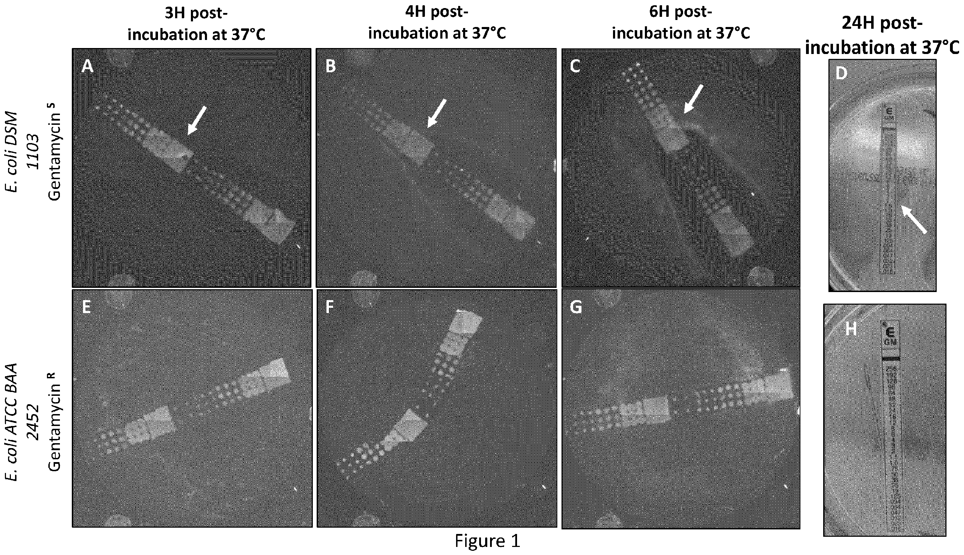

Figure 1 shows membrane images of *E. coli* DSM 1103 strain sensitive to Gentamycin or *E. coli* ATCC BAA 2452 strain resistant to gentamycin taken with a simple optical device at 3 hours (A and E), 4 hours (B and F) or 6 hours (C and G) respectively, after the start of the culture of each bacteria in the presence of antibiotic impregnated strips (white band on the images). Pictures D and H correspond to a 24 hours incubation of *E. coli* DSM 1103 strain and *E. coli* ATCC BAA 2452 strain respectively in presence of Gentamycin impregnated strips directly on MH agar plate. White arrows indicated the intersection between the inhibition ellipse and the strip that determines the MIC of the strain against Gentamycin.

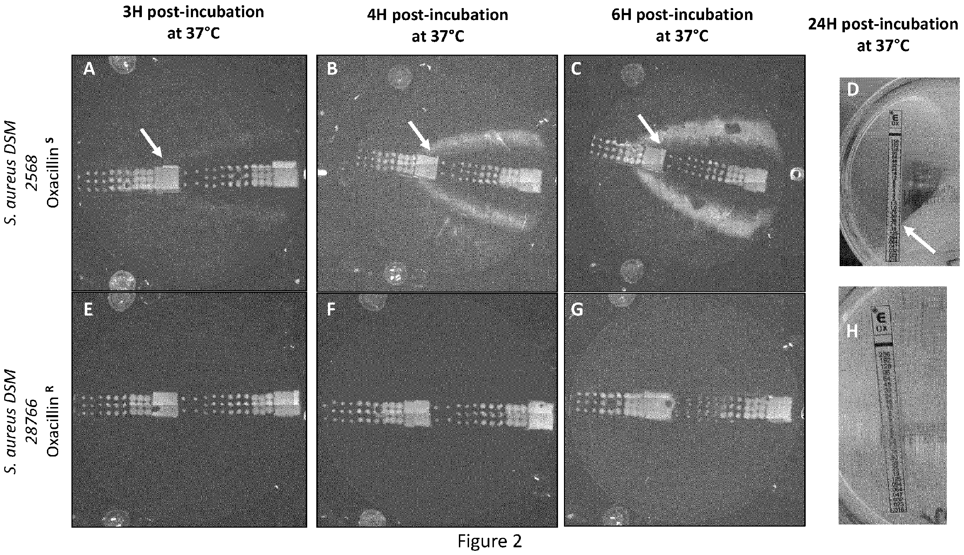

Figure 2 shows membrane images of S. *aureus* DSM 2568 strain sensitive to Oxacillin or S. *aureus* DSM 28766 strain resistant to Oxacillin taken with a simple optical device at 3 hours (A and E), 4 hours (B and F) or 6 hours (C and G) respectively, after the start of the culture of each bacteria in the presence of antibiotic impregnated strips (white band on the images). Pictures D and H correspond to a 24 hours incubation of S. *aureus* DSM 2568 strain and S. *aureus* DSM 28766 strain respectively in presence of Oxacillin impregnated strips directly on MH agar plate. White arrows indicated the intersection between the inhibition ellipse and the strip that determines the MIC of the strain against Oxacillin.

### DETAILED DISCUSSION OF PARTICULAR EMBODIMENTS

### Use of the method according to present invention for the evaluation of the MIC (minimum inhibitory concentration)

### A. Material and methods

### Samples

The following bacterial strains were used under the present study:
- *Escherichia coli* (sensitive to Gentamycin) - ref. DSM1103
- *Escherichia coli* (resistant to Gentamycin) - ref. ATCC^{®} BAA-2452^{™}
- *Staphylococcus aureus* (sensitive to Oxacillin) - DSMZ 2568
- *Staphylococcus aureus* (resistant to Oxacillin) - DSMZ 28766

### Description of resources and equipment used

### Equipment:

The equipment used comprises a filter bench, a rinsing bench and an optical bench.

The filter bench operates with a vacuum pump and can filter 6 samples in parallel.

The rinsing bench operates continuously with a pump and enables the simultaneous washing of 6 samples. This washing is performed in series, the wash solution flowing between the 6 samples and being recycled.

The optical bench is equipped with LEDs and filters enabling the reading of blue fluorescence ("DAPI" channel) or red fluorescence ("FM4-64" channel).

### Impregnated substrates:

The substrates used are Etest^{®} strips from BioMérieux, impregnated with, respectively, Gentamycin (for the experiments with E. coli) or Oxacillin (for the experiments with S. *aureus*). The references of the e-tests used can be found below:
- ETEST Gentamycin: ref.412368
- ETEST Oxacillin (= Methicillin): ref.412432

### Protocol

### J-1: Preparation of the bacterial samples

- inoculate the different strains of bacteria on TSA plates using the streak technique (one bacteria - one plate),
- incubate for 24h at 37°C.

### J0: AST: filtration or not, and incubation

➢**Protocol without a filtration step**
   - recover a few colonies of the same size and morphology in 2 mL of saline (0.9%NaCI),
   - adjust the MacFarland value to 0.5 using a nephelometer,
   - place a 45mm black MCE membrane with 0.45µm porosity (HABG047S6, Millipore) on the MH agar pre-incubated at 37°C, avoiding the formation of bubbles,
   - apply 1mL of MacFarland 0.5 suspension of the corresponding strain to the MCE membrane,
   - add 10 sterile glass beads 2 mm in diameter, and shake the box 10 times up and down, then 10 times from left to right, and finally 10 clockwise and 10 counter-clockwise rotations,
   - place the E-test with a plastic forceps avoiding the formation of bubbles to allow the good diffusion of the antibiotic in the agar,
   - incubation at 37°C for 3, 4 or 6 hours, so that the E-test agar is facing upwards.
➢ **Alternative protocol with a filtration step (not performed in the present case)**
   - recover a few colonies of the same size and morphology in 2 mL of saline (0.9%NaCI),
   - adjust the MacFarland value to 0.5 using a nephelometer,
   - deposition of the 45mm black MCE membrane with 0.45µm porosity (HABG047S6, Millipore) on the filtration ramp (Diamidex),
   - put the funnels,
   - prepare a solution of 4 mL of physiological water with 1 mL of bacteria at 0.5 MacFarland,
   - pour this 5mL solution into the funnel and filter the bacterial solution onto the membrane,
   - place the membrane on MH agar pre-incubated at 37°C,
   - place the E-test with a plastic forceps avoiding the formation of bubbles to allow the good diffusion of the antibiotic in the agar
   - incubation at 37°C for 3, 4 or 6 hours, so that the E-test agar is facing upwards.

### Marking

- after incubation, perform the DAPI labelling as follows:
   ∘ prepare a solution of DAPI at 1.4µg/mL, and filter at 0.22µm,
   ∘ place 700 µL of the DAPI solution at 1.4µg/mL on a glass fiber pad (GE Healthcare Whatman: GF/A ref. 1820-047; batch 17076495) in a petri dish,
   ∘ Put the membranes on the glass,
   ∘ incubate for 10 min at room temperature protected from light,
   ∘ wash at the rinsing bench (Diamidex) with 500mL phosphate buffer + 0.5 mL Tween20 (cfinal = 0.1%) for 15 min at room temperature in the dark. The membranes may be stored at 4°C after marking until the following day
- reading on the optical bench (Diamidex "model 2", magnification: 4 and resolution:
   12Mpix) is performed using the following parameters:
   - UV power: 10%
   - Exposure time: 262 ms
   - Gain = 1

### Results

For both *E. coli* (figure 1) and S. *aureus* (figure 2), differences of growth are observed between the resistant and sensitive strains from 3h of culture. Halos are observed around the strips and intensify with time of culture. The arrows in the figures indicate the minimum inhibitory concentration of the antibiotic for the bacterial strain tested.

### REFERENCES

[1] International application WO 2013/130875 A1 in the name of Harvard College, published on September 6, 2013.
**[2]** International application WO 2013/107759 A1 in the name of Centre National de la Recherche Scientifique, published on July 25, 2013.

## Claims

1. A method of determining the existence and/or degree of resistance of a microorganism to one or more antimicrobial agents, each at one or more concentrations, comprising:
a. forming a suspension of said microorganism in a liquid medium,
b. depositing homogeneously microorganism cells contained in said suspension on at least a part of the upper surface of a membrane,
c. laying said membrane by its lower surface on a culture medium,
d. placing one or more substrates each impregnated with one of said antimicrobial agents in contact with said membrane,
e. incubating the whole under conditions suitable for promoting the growth of said microorganism,
f. putting the lower surface of said membrane into contact with a fluorescent compound capable of labelling an endogenous constituent of said microorganism in order to label said microorganism cells,
g. optionally washing the lower surface of said membrane thereby eliminating the excess of fluorescent compound,
h. detecting the presence or absence of fluorescence around the substrate(s) on the upper surface of the membrane using a fluorescence-reading optical device,
i. and determining the existence and/or degree of resistance of said microorganism to said antimicrobial agent(s) based on the detection of presence and/or size of a non-fluorescent zone around said substrate(s).

2. The method according to claim 1, wherein said suspension has a McFarland value of between 0.4 and 0.6 and said incubating step is carried out for a duration of between 3 and 6 hours.

3. The method according to claim 1 or 2, wherein step d can be carried out before step b or before step c.

4. The method according to any of claims 1 to 3, wherein at least one of said substrates is a disk impregnated with a single concentration of one of said antimicrobial agents.

5. The method according to any of claims 1 to 3, wherein at least one of said substrates is a strip impregnated with a concentration gradient of one of said antimicrobial agent along its length.

6. The method according to any of the preceding claims, wherein a minimum inhibitory concentration (MIC) of the antimicrobial agent for the microorganism is determined.

7. The method according to any of the preceding claims, wherein said substrates are made of paper or plastic.

8. The method according to any of the preceding claims, wherein said optical device has a magnification between 1 and 50 and an optical resolution comprised between 6-20 Mpix.

9. The method according to any of the preceding claims, wherein a light source in said optical device comprises one or several LED(s).

10. The method according to any of the preceding claims, wherein at least one of said antimicrobial agents is an antibiotic.

11. The method according to any of the preceding claims, wherein said microorganism is selected from yeasts, fungi, algae, bacteria of the Gram positive type, bacteria of the Gram negative type, archaea and mixtures thereof.

12. The method according to any of the preceding claims, wherein said membrane is made of polytetrafluoroethylene (PTFE), polyester, polycarbonate, nylon, polyvinylidene fluoride (PVDF), cellulose and cellulose derivatives, such as cellulose acetate, cellulose nitrate, regenerated cellulose, nitrocellulose, cellophane, or mixed cellulose esters (MCE).

13. The method according to any of the preceding claims, wherein said fluorescent compound is capable of labelling mitochondria, cell membranes or nucleic acids.

14. The method according to any of the preceding claims, wherein said fluorescent compound is 4',6-diamidino-2-phenylindole (DAPI).

15. A method of analysing a sample for its microbial characteristics, comprising:
- detecting the presence of at least one microorganism in said sample,
- optionally, identifying said microorganism,
- recovering said microorganism from said sample,
- and performing on said recovered microorganism a method according to any of claims 1 to 14 of determining the existence and/or degree of resistance of a microorganism to one or more antimicrobial agents, each at one or more concentrations.

16. The method of claim 15, wherein said sample is a biological fluid; a plant fluid such as sap, nectar and root exudate; a sample in a culture medium or in a biological culture reactor such as a cell culture of higher eukaryotes, of yeasts, of fungi or of algae; a liquid obtained from one (or more) animal or plant cell(s); a liquid sample obtained from an animal or plant tissue; a liquid sample obtained from a food matrix; a liquid sample from a chemical reactor; municipal, river, pond, lake, sea, or air-cooled tower water; a sample from a liquid industrial effluent; waste water coming in particularfrom intensive animal production or industries of the chemical, pharmaceutical, cosmetic or nuclear field; a liquid sample from a pharmaceutical product; a liquid sample from a cosmetic product; a fragrance; a soil sample or a mixture thereof.
